(19) Europäisches Patentamt
European Patent Office
Office européen des brevets

(11) Veröffentlichungsnummer: **0 317 506**
**A2**

(12) # EUROPÄISCHE PATENTANMELDUNG

(21) Anmeldenummer: **88810754.7**

(22) Anmeldetag: **04.11.88**

(51) Int. Cl.⁴: **C 07 D 221/22**

(30) Priorität: **13.11.87 CH 4434/87**

(43) Veröffentlichungstag der Anmeldung:
**24.05.89 Patentblatt 89/21**

(84) Benannte Vertragsstaaten:
**CH DE FR GB IT LI NL**

(71) Anmelder: **CIBA-GEIGY AG**
**Klybeckstrasse 141**
**CH-4002 Basel (CH)**

(72) Erfinder: **Alder, Alex, Dr.**
**Häglerstrasse 22**
**CH-4422 Arisdorf (CH)**

(54) **Azabicycloheptane und Verfahren zu deren Herstellung.**

(57) Verbindungen der Formel I

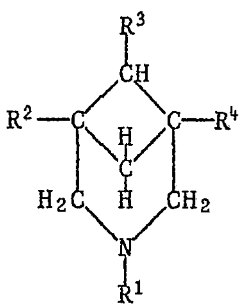

(I),

worin $R^1$, $R^2$, $R^3$ und $R^4$ z.B. H, Alkyl, Cycloalkyl, Aryl oder Alkaryl bedeuten, eignen sich als ein Ammoniumsalz bildende Komponente für saure, pharmazeutische Wirkstoffe und besonders zur Herstellung von Azabicyclo[3.1.1]heptan substituierten Alkandiphosphonsäuren zur Behandlung von auf Störungen des Calciumstoffwechsels zurückzuführenden Erkrankungen.

EP 0 317 506 A2

## Beschreibung

### Azabicycloheptane und Verfahren zu deren Herstellung

Die Erfindung betrifft substituierte 3-Azabicyclo[3.1.1]heptane und ein Verfahren zu deren Herstellung durch Reduktion von 3-Azabicyclo[3.1.1]heptan-2,4-dionen.

Ein Gegenstand der Erfindung sind Verbindungen der Formel I

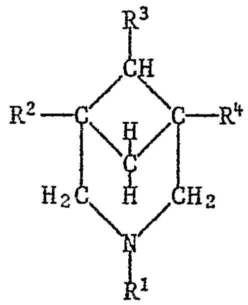

(I)

und ihre Ammoniumsalze, worin

$R^1$ ein Wasserstoffatom, lineares oder verzweigtes $C_1$-$C_{20}$-Alkyl, $C_3$-$C_8$-Cycloalkyl, $C_4$-$C_{20}$-Alkylcycloalkyl, $C_4$-$C_{12}$-Cycloalkylalkyl, $C_5$-$C_{20}$-Alkylcycloalkylalkyl, $C_6$-$C_{14}$-Aryl, $C_7$-$C_{20}$-Alkaryl oder -Aralkyl oder $C_8$-$C_{20}$-Alkaralkyl bedeutet, das unsubstituiert oder mit Halogen, -OH, -CN, $-NO_2$, $C_1$-$C_6$-Halogenalkyl, -COOH, $-SO_3H$, $R^5CO$-, $R^5COO$-, $R^5OCO$-, $R^5OSO_2$-, $R^5SO_2$-, $R^5SO_3$-, $C_1$-$C_6$-Halogenalkyl, $C_1$-$C_6$-Alkoxy, $C_1$-$C_6$-Alkylthio, $C_6$-$C_{10}$-Aryloxy oder -Arylthio, $C_7$-$C_{12}$-Aralkyloxy oder -Aralkylthio substituiert ist, wobei $R^5$ $C_1$-$C_{12}$-Alkyl, $C_4$-$C_7$-Cycloalkyl, $C_5$-$C_{16}$-Alkylcycloalkyl oder -Cycloalkylalkyl, $C_6$-$C_{16}$-Alkylcycloalkylalkyl, $C_6$-$C_{10}$-Aryl, $C_7$-$C_{16}$-Aralkyl oder -Alkaryl oder $C_8$-$C_{16}$-Alkaralkyl ist,

$R^2$ für ein Wasserstoffatom, lineares oder verzweigtes $C_1$-$C_{20}$-Alkyl, -Alkoxy, -Alkylthio, $C_3$-$C_8$-Cycloalkyl, $C_4$-$C_{20}$-Alkylcycloalkyl oder -Cycloalkylalkyl, $C_5$-$C_{20}$-Alkylcycloalkylalkyl, $C_3$-$C_8$-Cycloalkyloxy oder -thio, $C_4$-$C_{20}$-Alkylcycloalkyloxy oder -thio oder -Cycloalkyl alkyloxy oder -thio, $C_5$-$C_{20}$-Alkylcycloalkylalkyloxy oder -thio, $C_6$-$C_{14}$-Aryl, -Aryloxy oder -thio, $C_7$-$C_{20}$-Alkaryl, -Alkaryloxy oder -thio, $C_7$-$C_{20}$-Aralkyl, $C_7$-$C_{20}$-Aralkyloxy oder -thio, $C_8$-$C_{20}$-Alkaralkyl, $C_8$-$C_{20}$-Alkaralkyloxy oder -thio steht, das unsubstituiert oder wie für $R^1$ definiert, substituiert ist,

$R^4$ unabhängig die gleiche Bedeutung wie $R^2$ hat mit Ausnahme von unsubstituiertem, oder wie für $R^1$ definiert substituiertem $C_6$-$C_{14}$-Aryl und $C_7$-$C_{20}$-Alkaryl, und

$R^3$ unabhängig die Bedeutung von $R^2$ hat, oder Halogen, Carboxyl oder Carboxylat mit 2 bis 16 C-Atomen darstellt, mit der Massgabe, dass $R^1$, $R^2$, $R^3$ und $R^4$ nicht gleichzeitig für H stehen.

$R^1$, $R^2$, $R^3$ und $R^4$ können ein- oder mehrfach, vorzugsweise ein- bis fünffach, besonders ein- bis dreifach und insbesondere ein- oder zweifach substituiert sein. Geeignete Substituenten sind z.B.: Halogen, besonders F, Cl, Br; -OH, -CN, $-NO_2$, -COOH, $SO_3H$; $R^5CO$-, $R^5COO$-, $R^5OCO$-, $R^5SO_2$-, $R^5OSO_2$- und $R^5SO_3$-, worin $R^5$ vorzugsweise für lineares oder verzweigtes $C_1$-$C_6$-, besonders $C_1$-$C_4$-Alkyl, Cyclohexyl, Phenyl, $C_1$-$C_4$-Alkylphenyl, Benzyl oder $C_1$-$C_4$-Alkylbenzyl steht;

$C_1$-$C_6$-, bevorzugt $C_1$-$C_4$-Halogenalkyl, worin das Halogen besonders F oder Cl ist, z.B. Chlormethyl, Fluormethyl, Trifluormethyl, 2-Fluorethyl, 2-Chlorethyl, 1,1-Difluoreth-1-yl, 2,2,2-Trifluoreth-1-yl; $C_1$-$C_6$-, bevorzugt $C_1$-$C_4$-Alkoxy oder -Alkylthio, z.B. Methoxy, Ethoxy, Propyloxy, Butyloxy, Methylthio und Ethylthio; $C_6$-$C_{10}$-Aryloxy oder -Arylthio, besonders Phenoxy und Phenylthio; $C_7$-$C_{12}$-Aralkyloxy oder -Aralkylthio, besonders Benzyloxy oder Benzylthio.

Die Kohlenwasserstoffreste der Substituenten können ihrerseits ein-oder mehrfach, besonders ein- oder zweifach substituiert sein, z.B. mit $C_1$-$C_4$-Alkyl oder -Alkoxy, F, Cl, Br, -OH oder -CN.

Bei $R^1$, $R^2$, $R^3$ und $R^4$ kann es sich um lineares oder verzweigtes Alkyl mit vorzugsweise 1 bis 12, besonders 1 bis 6 C-Atomen handeln. Beispiele sind Methyl, Ethyl und die Isomeren von Propyl, Butyl, Pentyl, Hexyl, Heptyl, Octyl, Nonyl, Decyl, Undecyl, Dodecyl, Tetradecyl, Hexadecyl, Octadecyl und Eicosyl.

$R^1$, $R^2$, $R^3$ und $R^4$ können Cycloalkyl mit bevorzugt 5 bis 7, besonders 5 oder 6 Ring-C-Atomen, z.B. Cyclopentyl und Cyclohexyl, sein.

$R^1$, $R^2$, $R^3$ und $R^4$ können Alkylcycloalkyl oder -Cycloalkylalkyl mit bevorzugt 6 bis 16 C-Atomen sein. Bei dem Cycloalkylrest handelt es sich bevorzugt um Cyclopentyl oder Cyclohexyl. Besonders bevorzugt ist $C_1$-$C_4$-Alkylcycloalkyl, z.B. Methyl- oder Ethylcyclohexyl und Cycloalkylmethyl, z.B. Cyclohexylmethyl.

$R^1$, $R^2$, $R^3$ und $R^4$ können Alkylcycloalkylalkyl mit bevorzugt 7 bis 16 C-Atomen sein. Der Cycloalkylrest enthält bevorzugt 5 oder 6 Ring-C-Atome. Besonders bevorzugt ist $C_1$-$C_4$-Alkylcycloalkylmethyl, z.B. (Methylcyclohexyl)methyl oder (Ethylcyclohexyl)methyl.

$R^1$, $R^2$ und $R^3$ können $C_6$-$C_{14}$-, besonders $C_6$-$C_{10}$-Aryl oder $C_7$-$C_{20}$-, bevorzugt $C_7$-$C_{16}$-Alkaryl sein. Bei dem Arylrest handelt es sich bevorzugt um einen Naphthyl- und besonders Phenylrest. Besonders bevorzugt

sind Phenyl und $C_1$-$C_4$-Alkylphenyl. $R^1$, $R^2$, $R^3$ und $R^4$ können $C_7$-$C_{20}$-, bevorzugt $C_7$-$C_{16}$-Aralkyl, oder $C_8$-$C_{20}$-, bevorzugt $C_8$-$C_{16}$-Alkaralkyl sein. Bei dem Arylrest handelt es sich bevorzugt um Naphthyl und besonders um Phenyl. Besonders bevorzugt sind Phenyl-$C_nH_{2n}$- und $C_1$-$C_4$-Alkylphenyl-$C_nH_{2n}$-, worin n eine Zahl von 1 bis 4, besonders 1 oder 2 ist. Bevorzugt ist Benzyl und $C_1$-$C_4$-Alkylbenzyl.

Bedeuten $R^2$, $R^3$ und $R^4$ Oxy- oder Thioreste, so gelten für diese Reste die gleichen Bevorzugungen wie für die entsprechenden Kohlenwasserstoffreste.

Bei $R^3$ kann es sich auch um Halogen, besonders F, Cl und Br, sowie Carboxyl oder $C_2$-$C_{16}$-, besonders $C_2$-$C_8$-Carboxylat handeln. In der Bedeutung von Carboxylat handelt es sich besonders um -COO-$C_1$-$C_4$-Alkyl.

Bei den Ammoniumsalzen handelt es sich besonders um Ammoniumhydrosalze von Mineralsäuren und organischen Säuren. Solche Säuren sind z.B. Halogenwasserstoffsäuren (Salzsäure, Bromwasserstoffsäure, Jodwasserstoffsäure), Kohlensäure, Schwefelsäure, Phosphorsäure, $C_1$-$C_8$-Carbonsäuren (Ameisensäure, Essigsäure, Propionsäure, Trichlor- oder Trifluoressigsäure, Benzoesäure, Phenylessigsäure) und $C_1$-$C_8$-Sulfonsäuren (Methansulfonsäure, Trifluormethansulfonsäure, Phenylsulfonsäure, p-Toluolsulfonsäure).

In einer bevorzugten Ausführungsform ist $R^1$ ein Wasserstoffatom oder unsubstituiertes oder substituiertes $C_1$-$C_{12}$-Alkyl, $C_5$-$C_7$-Cycloalkyl, $C_6$-$C_{16}$-Alkylcycloalkyl oder -Cycloalkylalkyl, $C_7$-$C_{16}$-Alkylcycloalkylalkyl, $C_6$-$C_{10}$-Aryl, $C_7$-$C_{16}$-Aralkyl oder -Alkaryl oder $C_8$-$C_{16}$-Alkaralkyl.

In einer anderen bevorzugten Ausführungsform ist $R^1$ ein Wasserstoffatom oder unsubstituiertes oder substituiertes $C_1$-$C_6$-Alkyl, Cyclopentyl, Cyclohexyl, $C_1$-$C_4$-Alkylcyclopentyl oder -cyclohexyl, Cyclopentyl-$C_nH_{2n}$- oder Cyclohexyl-$C_nH_{2n}$-, $C_1$-$C_4$-Alkylcyclopentyl-$C_nH_{2n}$- oder $C_1$-$C_4$-Alkylcyclohexyl-$C_nH_{2n}$-, Phenyl, $C_1$-$C_4$-Alkylphenyl, Phenyl-$C_nH_{2n}$-, $C_1$-$C_4$-Alkylphenyl-$C_nH_{2n}$-, wobei n eine Zahl von 1 bis 4, besonders 1 oder 2 darstellt.

Besonders bevorzugt ist $R^1$ H, unsubstituiertes oder substituiertes $C_1$-$C_6$-Alkyl, Phenyl oder Benzyl.

In einer anderen Ausführungsform ist $R^2$ H, oder unsubstituiertes oder substituiertes $C_1$-$C_{12}$-Alkyl, -Alkoxy oder -Alkylthio, $C_5$-$C_7$-Cycloalkyl, $C_6$-$C_{16}$-Alkylcycloalkyl oder -Cycloalkylalkyl, $C_7$-$C_{16}$-Alkylcycloalkylalkyl, $C_5$-$C_7$-Cycloalkoxy oder -thio, $C_6$-$C_{16}$-Alkylcycloalkyloxy oder -thio oder -Cycloalkylalkyloxy oder -thio, $C_7$-$C_{16}$-Alkylcycloalkylalkyloxy oder -thio, $C_6$-$C_{10}$-Aryl, -Aryloxy oder -thio, $C_7$-$C_{16}$-Alkaryl, -Alkaryloxy oder -thio oder -Aralkyloxy oder -thio, $C_8$-$C_{16}$-Alkaralkyloxy oder -thio, $C_7$-$C_{16}$-Alkaryl oder $C_8$-$C_{16}$- Alkaralkyl. Für $R^4$ gelten die gleichen Bevorzugungen, mit Ausnahme von Aryl und Alkaryl.

In einer anderen bevorzugten Ausführungsform steht $R^2$ für H oder unsubstituiertes oder substituiertes $C_1$-$C_6$-, besonders $C_1$-$C_4$-Alkyl, Phenyl oder $C_1$-$C_4$-Alkylphenyl. Insbesondere ist $R^2$ H, $C_1$-$C_4$-Alkyl oder unsubstituiertes oder substituiertes Phenyl oder $C_1$-$C_4$-Alkylphenyl.

$R^4$ steht insbesondere für H oder $C_1$-$C_6$-, besonders für $C_1$-$C_4$-Alkyl.

Eine bevorzugte Ausführungsform ist jene, worin $R^3$ für H steht, oder unsubstituiertes oder substituiertes $C_1$-$C_{12}$-Alkyl, -Alkoxy oder -Alkylthio, $C_5$-$C_7$-Cycloalkyl, $C_6$-$C_{16}$-Alkylcycloalkyl oder -Cycloalkylalkyl, $C_7$-$C_{16}$-Alkylcycloalkylalkyl, $C_5$-$C_7$-Cycloalkoxy oder -thio, $C_6$-$C_{16}$-Alkylcycloalkyloxy oder -thio oder -Cycloalkylalkyloxy oder -thio, $C_7$-$C_{16}$-Alkylcycloalkylalkyloxy oder -thio, $C_6$-$C_{10}$-Aryl, -Aryloxy oder -thio, $C_7$-$C_{16}$-Alkaryl, -Alkaryloxy oder -thio oder -Aralkyloxy oder -thio, $C_8$-$C_{16}$-Alkaralkyloxy oder -thio, $C_7$-$C_{16}$-Alkaryl oder $C_8$-$C_{16}$-Alkaralkyl, F, Cl, Br, Carboxyl oder Carboxylat mit 2 bis 8 C-Atomen darstellt.

Besonders bevorzugt ist $R^3$ H, $C_1$-$C_6$-Alkyl, unsubstituiertes oder substituiertes Phenyl oder $C_1$-$C_4$-Alkylphenyl.

In einer besonders bevorzugten Ausführungsform ist $R^4$ H und $R^2$ Phenyl, oder $R^2$ und $R^4$ Methyl und $R^1$ und $R^3$ H.

Ein weiterer Gegenstand vorliegender Erfindung ist ein Verfahren zur Herstellung von Verbindungen der Formel I, das dadurch gekennzeichnet ist, dass man eine Verbindung der Formel II

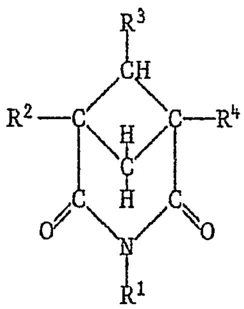

(II),

worin $R^1$, $R^2$, $R^3$ und $R^4$ die in Anspruch 1 angegebenen Bedeutungen haben, reduziert.

Die Verbindungen der Formel II sind teilweise bekannt oder sie können in analoger Weise durch photochemische oder thermische [2+2]-Cycloaddition von N-substituierten Diacrylimiden hergestellt werden. Solche Verfahren sind z.B. in Helv.Chim.Acta., Vol. 65, Fasc. 8, S. 2405-2412 (1982) und im J. of Am. Chem. Soc., Vol. 105, S. 6712-6714 (1983) beschrieben.

Die Reduktion kann elektrochemisch, katalytisch oder mit Metallhydriden vorgenommen werden. Geeignete Metallhydride sind z.B. LiH, $LiAlH_4$, Tributylzinnhydrid und besonders bisalkoxylierte Natriumdihydroaluminate. Besonders bevorzugt ist Natriumdihydro-bis-(2-methoxyethoxy)-aluminat.

Die Reduktion wird zweckmässig in Gegenwart eines inerten Lösungsmittels durchgeführt. Geeignete Lösungsmittel sind z.B. Ether (Diethylether, Dibutylether, Tetrahydrofuran oder Dioxan) oder Kohlenwasserstoffe wie z.B. Petrolether, Pentan, Methylcyclohexan, Benzol, Toluol oder Xylol.

Im einzelnen kann man so vorgehen, dass man die Verbindung der Formel II mit dem Lösungsmittel vorlegt und eine Lösung des Reduktionsmittels langsam zugibt. Vorteilhaft wird unter Inertgasatmosphäre gearbeitet, z.B. Stickstoff oder einem Edelgas wie z.B. Helium, Neon oder Argon. Während der Zugabe des Reduktionsmittels kann eine externe Kühlung zweckmässig sein, um die Reaktionstempe ratur niedrig zu halten. Nach Beendigung der Zugabe kann das Reaktionsgemisch noch einige Zeit weitergerührt werden, gegebenenfalls unter Erwärmen bis auf Rückflusstemperatur.

Zur Isolierung wird das Reaktionsgemisch zweckmässig hydrolysiert, z.B. mit wässrigen Alkalimetallhydroxiden, die organische Phase abgetrennt, gewaschen, getrocknet, und das Lösungsmittel abdestilliert. Die Reinigung kann durch Destillation oder chromatographische Methoden erfolgen. Besonders zweckmässig ist es, die cyclischen Amine der Formel I in einem Ether, z.B. Diethylether zu lösen und anschliessend durch Einleiten eines Halogenwasserstoffgases, z.B. HCl-Gas, als kristalline Hydrohalogenide auszufällen und gegebenenfalls durch Umkristallisation weiter zu reinigen.

Die Amine der Formel I bzw. ihre Salze eignen sich als salzbildende Komponente für pharmazeutische Wirkstoffe mit sauren Gruppen. Insbesondere eignen sich die erfindungsgemässen Verbindungen zur Herstellung von Arzneimittelwirkstoffen für die Behandlung von Erkrankungen, die mit Störungen des Calciumstoffwechsels in Verbindung gebracht werden können. Diese Wirkstoffe können der Formel A

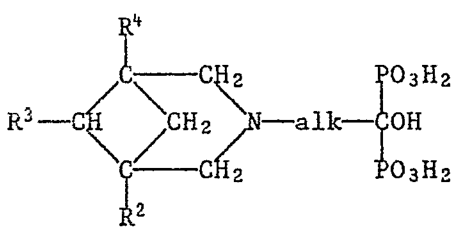

(A)

entsprechen, wobei auch Salze, z.B. das Dinatriumsalz, verwendbar sind. Alk kann hierin die Bedeutung von Alkylen mit bevorzugt 2 bis 6 C-Atomen haben. Diese Verbindungen können z.B. hergestellt werden, indem man eine Verbindung der Formel B

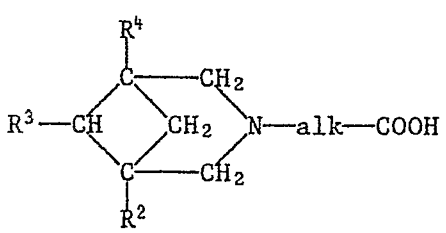

(B)

oder deren Hydrochloride mit konzentrierter Phosphorsäure und Phosphortrichlorid umsetzt, anschliessend mit Chlorwasserstoffsäure behandelt und dann die Verbindungen der Formel (A) isoliert. Die Verbindungen der Formel (B) sind erhältlich, indem man eine Verbindung der Formel I, worin $R^1$ H bedeutet, an olefinisch ungesättigte aliphatische Carbonsäureester anlagert und den erhaltenen Ester hydrolysiert.

Die Verbindungen der Formel A und ihre Salze weisen wertvolle pharmakologische Eigenschaften auf. Insbesondere besitzen sie eine ausgeprägte regulierende Wirkung auf den Calcium-Stoffwechsel von Warmblütern. Insbesondere bewirken sie an der Ratte eine ausgeprägte Hemmung der Knochenresorption, die sich sowohl in der Versuchsanordnung gemäss Acta Endocrinol. 78, 613-24 (1975) anhand des PTH-induzierten Anstieges des Serumcalciumspiegels nach subcutaner Applikation in Dosen von etwa 0,01 bis etwa 1,0 mg/kg, als auch im TPTX (Thyroparathyroidectomised) - Rattenmodell anhand der durch Vitamin $D_3$ ausgelösten experimentellen Hypercalcämie nach Gabe von Dosen von etwa 0,001 bis 1.0 mg s.c. zeigen lässt. Ebenso wird die durch Walker-256-Tumore induzierte Tumorhyperkalzämie nach peroraler Verabreichung von etwa 1,0 bis etwa 100 mg/kg gehemmt. Ferner zeigen sie in der Adjuvansarthritis der Ratte in der Versuchsanordnung nach Newbould, Brit. J. Pharmacology 21, 127 (1963) sowie nach Kaibara et al., J. Exp.

Med. 159, 1388-96 (1984) in Dosen von etwa 0,01 bis 1,0 mg/kg s.c. eine deutliche Hemmung auf das Fortschreiten chronisch-arthritischer Prozesse.

Die nachfolgenden Beispiele erläutern die Erfindung näher.

Beispiel 1:

a) 1-Phenyl-3-azabicyclo[3.1.1]heptan-hydrochlorid

Zur gerührten Suspension von 3 g 1-Phenyl-3-azabicyclo[3.1.1.]heptan-2,4-dion in 150 ml Toluol werden unter Stickstoffatmosphäre 25,5 ml Natriumdihydro-bis-(2-methoxyethoxy)-aluminat-Toluollösung (70 %; FLUKA) zugetropft. Während der Zugabe wird die Temperatur durch externe Kühlung im Eisbad im Bereich von 25-35° C gehalten. Nach beendeter Zugabe wird 15 Minuten bei Raumtemperatur (RT) nachgerührt und anschliessend 1 Stunde unter Rückfluss erwärmt. Nach dem Abkühlen im Eisbad werden bei 10-15° C 25,5 ml konzentrierte Natronlauge zugetropft. Die organische Phase wird abdekantiert, und die Wasserphase mit Toluol gewaschen. Die vereinigten organischen Phasen werden zweimal mit 100 ml Wasser und einmal mit 70 ml Sole gewaschen. Nach der Zugabe von Magnesiumsulfat wird die organische Phase filtriert und am Wasserstrahlvakuum eingeengt. Das bräunliche Oel wird in 50 ml absolutem Diethylether gelöst. Die Titelverbindung wird durch Einleiten von HCl-Gas als kristallines Produkt erhalten, welches nach dem Abnutschen nochmals in Diethylether aufgeschlämmt und wiederum abgenutscht und zuletzt am Hochvakuum über Nacht getrocknet wird. Man erhält weisse Kristalle mit einem Schmelzpunkt (Smp.) von 248-249° C.

Herstellung des Ausgangsmaterials:

b) 1-Phenyl-3-azabicyclo[3.1.1]heptan-2,4-dion

Zu einer gerührten Lösung von 53 g 3-(4-Methoxybenzyl)-1-phenyl-3-azabicyclo[3.1.1]heptan-2,4-dion in 560 ml Acetonitril wird bei Raumtemperatur eine Lösung von 344 g Cer(IV)-ammoniumnitrat in 1,1 l Acetonitril zugetropft. Nach 1 Stunde werden 515 ml Wasser zugegeben, und es wird während 2 Stunden gerührt. Durch Abdestillieren von Acetonitril wird auf das halbe Volumen eingeengt und anschliessend mit 1 l Wasser verdünnt. Das ausgefallene Produkt wird abgenutscht, mit Wasser gewaschen und am Vakuum getrocknet. Die braungelbliche kristalline Substanz wird in 800 ml Methylenchlorid aufgenommen, mit 11 ml n-Propylamin versetzt und über Nacht stehen gelassen. Die schwarze Lösung wird eingeengt und die braune kristalline Masse mit 80 ml Methylenchlorid-Diethylether (1:1) versetzt, abgenutscht und am Hochvakuum getrocknet. Man erhält die Titelverbindung als weisse Kristalle mit einem Smp. von 217-218° C.

c) 3-(4-Methoxybenzyl)-1-phenyl-3-azabicyclo[3.1.1]heptan-2,4-dion

Eine Lösung von 65,7 g 4-Aza-4-(4-methoxybenzyl)-2-phenyl-1,6-heptadien-3,5-dion und 0,5 g 2,6-Di-tert.butyl-p-kresol in 1 l 1,3-Dichlorbenzol wird während 6 Stunden bei 170° C gerührt. Nach dem Eindampfen wird der Rückstand mit Toluol-Diethylether (9:1) an 2,5 kg Kieselgel 60 chromatographiert. Das so erhaltene braune Oel wird in 550 ml Diisopropylether bei 70° C gelöst und unter Rühren im Eisbad abgekühlt. Der Niederschlag wird abgenutscht und man erhält nach dem Trocknen am Hochvakuum die Titelverbindung als weisse Kristalle mit einem Smp. von 87-88° C.

d) 4-Aza-4-(4-methoxybenzyl)-2-phenyl-1,6-heptadien-3,5-dion

Zu einer gerührten Lösung von 88,8 g 2-Phenylacrylsäure in 7 ml Dimethylformamid und 1,6 l Methylenchlorid wird während 2 1/2 Stunden bei Raumtemperatur eine Lösung von 103 ml Oxalylchlorid in 400 ml Methylenchlorid getropft. Es wird nach beendeter Zugabe noch 2 Stunden gerührt und anschliessend am Vakuum eingedampft. Das braune ölige Produkt wird in 600 ml Diethylether aufgenommen und vom klebrigen Rückstand getrennt, über HYFLO-Super-Cel® filtriert und am Vakuum eingeengt. Das braune Oel wird in 0,8 l Methylenchlorid gelöst und in eine auf 0-5° C gekühlte Lösung von 95,6 g N-(4-Methoxybenzyl)-acrylamid, 6,43 g 4-Dimethylaminopyridin und 63,1 g Triethylamin in 1 l Methylenchlorid getropft. Nach beendeter Zugabe wird 3 Stunden bei Raumtemperatur nachgerührt. Nach dem Eindampfen der Reaktionslösung auf 250 ml wird 1 l Diethylether zugegeben. Die organische Phase wird vom klebrigen Rückstand abdekantiert. Der Rückstand wird dreimal mit 500 ml Diethylether aufgenommen und die organische Phase jeweils abdekantiert. Die vereinigten organischen Phasen werden auf 200 ml eingeengt und über HYFLO-Super-Cel® filtriert. Durch Einengen erhält man die Titelverbindung als braunes Oel (wird sofort gemäss 1c) weiter umgesetzt).

Beispiel 2:

a) 3-Methyl-1-phenyl-3-azabicyclo[3.1.1]heptan-hydrochlorid

Analog Beispiel 1a) werden 1 g 3-Methyl-1-phenyl-3-azabicyclo[3.1.1]heptan-2,4-dion in 25 ml Toluol mit 7,4 ml Natriumdihydro-bis-(2-methoxyethoxy)-aluminat-Toluollösung (70 %; FLUKA) umgesetzt und mit 7,4 ml konzentrierter Natronlauge aufgearbeitet. Smp. der Titelverbindung: 204-207° C.

Herstellung des Ausgangsmaterials:

b) 3-Methyl-1-phenyl-3-azabicyclo[3.1.1]heptan-2,4-dion

Eine Lösung von 50 g 4-Aza-4-methyl-2-phenyl-1,6-heptadien-3,5-dion, 1,2 g Benzophenon und 0,5 g 2,6-Di-tert.butyl-p-kresol in 2,5 l Methylenchloridaceton (1:1) wird während 48 Stunden mit einer in einem wassergekühlten Pyrexfinger angebrachten Quecksilberdampflampe (HPK 125W; Philips) bestrahlt. Nach dem Eindampfen wird das Rohprodukt mit Toluol-Essigester (9:1) an Kieselgel 60 chromatographiert. Das so erhaltene kristalline Produkt wird aus Diisopropylether-Methylenchlorid umkristallisiert: Smp. 175-176° C.

c) 4-Aza-4-methyl-2-phenyl-1,6-heptadien-3,5-dion

Zu einer gerührten, auf 0-5° C gekühlten Lösung von 35,6 g N-Methylacrylamid, 89,3 g Triethylamin und 350 ml Methylenchlorid wird eine analog zu 1d) hergestellte Lösung von 2-Phenylacrylsäurechlorid (ausgehend von 61,9 g 2-Phenylacrylsäure, 54,3 g Oxalylchlorid und 4 ml DMF in 350 ml Methylenchlorid) getropft. Nach beendeter Zugabe wird über Nacht bei Raumtemperatur gerührt. Nach dem Eindampfen der Reaktionslösung auf ca. 200 ml wird ca. 0,5 l Diethylether zugegeben, filtriert, eingeengt und über Hyflo-Super-Cel® filtriert. Nach dem Einengen wird das Produkt als dunkeloranges Oel erhalten (wird sofort gemäss 2b) weiter umgesetzt).

Beispiel 3:

a) 3-Methyl-1-(4-methylphenyl)-3-azabicyclo[3.1.1]heptan-hydrochlorid

Analog Beispiel 1a) werden 3,4 g 3-Methyl-1-(4-methylphenyl)-3-azabicyclo[3.1.1]heptan-2,4-dion in 75 ml Toluol mit 14 ml Natriumdihydro-bis-(2-methoxyethoxy)-aluminat-Toluollösung (70 %; FLUKA) umgesetzt und mit 14 ml konzentrierter Natronlauge aufgearbeitet. Smp. der Titelverbindung: 192-193° C.

Herstellung des Ausgangsmaterials

b) 3-Methyl-1-(4-methylphenyl)-3-azabicyclo[3.1.1.]heptan-2,4-dion

Eine Lösung von 19,9 g 4-Aza-4-methyl-2-(4-methylphenyl)-1,6-heptadien-3,5-dion und 0,2 g 2,6-Di-tert.butyl-p-kresol in 0,5 l Xylol wird während 19 Stunden am Rückfluss gerührt. Nach dem Einengen der Reaktionsmischung wird in 0,8 l Diisopropylether heiss gelöst, filtriert und zur Hälfte eingeengt. Beim Abkühlen auf Raumtemperatur erhält man die Titelverbindung als blassgelbe Kristalle mit einem Smp. von 138-140° C.

c) 4-Aza-4-methyl-1-(4-methylphenyl)-1,6-heptadien-3,5-dion

Zu einer gerührten, auf 0-5° C gekühlten Lösung von 11 g N-Methylacrylamid, 35 g Triethylamin und 125 ml Methylenchlorid wird eine analog zu 1d) hergestellte Lösung von 2-(4-Methylphenyl)-acrylsäurechlorid (ausgehend von 25,5 g 2-(4-Methylphenyl)-acrylsäure, 22 g Oxalylchlorid, 2 ml Dimethylformamid und 0,5 g Hydrochinon in 590 ml Methylenchlorid) getropft. Nach beendeter Zugabe wird 24 Stunden bei Raumtemperatur nachgerührt. Das eingeengte Reaktionsgut wird mit Methylenchlorid an 800 g Kieselgel 60 filtriert und man erhält nach dem Einengen die Titelverbindung als dunkeloranges Oel (wird sofort gemäss 3b) weiter umgesetzt).

Beispiel 4:

a) 1-(4-Chlorphenyl)-3-methyl-3-azabicyclo[3.1.1.]heptan-hydrochlorid

Analog Beispiel 1a) werden 3,9 g 1-(4-Chlorphenyl)-3-methyl-3-azabicyclo[3.1.1]heptan-2,4-dion in 70 ml Toluol mit 25 ml Natriumdihydro-bis-(2-methoxyethoxy)-aluminat-Toluollösung (70 %; FLUKA) umgesetzt und mit 25 ml konzentrierter Natronlauge aufgearbeitet. Smp. der Titelverbindung: 180-182° C.

Herstellung des Ausgangsmaterials

b) (1-(4-Chlorphenyl)-3-methyl-3-azabicyclo[3.1.1]heptan-2,4-dion

Eine Lösung von 28,5 g 4-Aza-2-(4-chlorphenyl)-4-methyl-1,6-heptadien-3,5-dion und 0,25 g 2,6-Di-tert.butyl-p-kresol in 1,25 l Methylenchlorid-Aceton (1:1) wird während 24 Stunden analog zu 2b) bestrahlt. Nach dem Einengen wird das Rohprodukt mit Toluol-Diethylether (9:1) an Kieselgel 60 chromatographiert. Man erhält die Titelverbindung nach Umkristallisation aus Chloroform-Diethylether mit einem Smp. von 155-156° C.

c) 4-Aza-2-(4-chlorphenyl)-4-methyl-1,6-heptadien-3,5-dion

Zu einer gerührten, auf 0-5° C gekühlten Lösung von 12 g N-Methylacrylamid, 31,6 g Triethylamin und 130 ml Methylenchlorid wird eine analog zu 1d) hergestellte Lösung von 2-(4-Chlorphenyl)-acrylsäurechlorid (ausgehend von 28,8 g 2-(4-Chlorphenyl)-acrylsäure, 19,9 g Oxalylchlorid, 1,5 ml Dimethylformamid in 750 ml Methylenchlorid) getropft. Nach beendeter Zugabe wird über Nacht bei Raumtemperatur gerührt. Es wird wie in 1c) aufgearbeitet und man erhält die Titelverbindung als braunes Oel, welches sofort gemäss (4b) weiter umgesetzt wird.

Beispiel 5:

3(4-Methoxybenzyl)-1-phenyl-3-azabicyclo[3.1.1]heptan-hydrochlorid

Analog Beispiel 1a) werden 2 g 3-(4-Methoxybenzyl)-1-phenyl-3-azabicyclo[3.1.1]heptan-2,4-dion (Herstellung s. Beispiel 1c) in 50 ml Toluol mit 5,5 ml Natriumdihydro-bis-(2-methoxyethoxy)-aluminat-Toluollösung (70 %; FLUKA) umgesetzt und mit 5,5 ml konzentrierter natronlauge aufgearbeitet. Smp. der Titelverbindung: 209-211°C.

Beispiel 6:

a) exo-6-(4-Chlorphenyl)-3-azabicyclo[3.1.1]heptan-hydrochlorid

Analog Beispiel 1a) werden 3,5 g exo-6-(4-Chlorphenyl)-3-azabicyclo[3.1.1]heptan-2,4-dion in 75 ml Toluol mit 24 ml Natriumdihydro-bis-(2-methoxyethoxy)-aluminat-Toluollösung (70 %; FLUKA) umgesetzt und mit 24 ml konzentrierter Natronlauge aufgearbeitet. Smp. der Titelverbindung: 201-203°C.

Herstellung des Ausgangsmaterials

b) exo-6-(4-Chlorphenyl)-3-azabicyclo[3.1.1]heptan-2,4-dion

Zu einer Lösung von 58,5 g exo-1-Brom-6-(4-chlorphenyl)-3-azabicyclo[3.1.1]heptan-2,4-dion und 1,9 g Bisazoisobutyronitril in 1 l absolutem Tetrahydrofuran werden unter Stickstoffatmosphäre 60 g Tri-n-butylzinnhydrid gegeben. Die Reaktionslösung wird 4 Stunden am Rückfluss erwärmt und anschliessend am Vakuum eingeengt. Der kristalline Rückstand wird in Cyclohexan aufgenommen, vermischt, abgenutscht und mit Diethylether gewaschen. Man erhält die Titelverbindung als weisse Kristalle mit einem Smp. von 182-183°C.

c) exo-1-Brom-6-(4-chlorphenyl)-3-azabicyclo[3.1.1]heptan-2,4-dion

Zu einer gerührten Suspension von 52,1 g exo-1-Brom-6-(4-chlorphenyl)-3-(4-methoxybenzyl)-3-azabicyclo[3.1.1]heptan-2,4-dion in 2,1 l Acetonitril wird bei Raumtemperatur eine Lösung von 249 g Cer(IV)-ammoniumnitrat in 330 ml Wasser zugetropft. Man rührt nach beendeter Zugabe noch 1 Stunde bei Raumtemperatur, erwärmt auf 40°C, lässt die Lösung langsam wieder auf Raumtemperatur erkalten und rührt weitere 3 Stunden. Es wird auf ein Drittel des Volumens eingeengt und danach mit 1,6 l Wasser verdünnt. Das ausgefallene Produkt wird abgenutscht, mit Wasser, Diethylether und Essigsäureethylester gewaschen und am Vakuum getrocknet. Man erhält die Titelverbindung als gelbe Kristalle mit einem Smp. von 231-232°C.

d) exo-1-Brom-6-(4-chlorphenyl)-3-(4-methoxybenzyl)-3-azabicyclo[3.1.1]heptan-2,4-dion

Eine Lösung von 111,8 g 4-Aza-2-brom-7-(4-chlorphenyl)-4-(4-methoxybenzyl)-1,6-heptadien-3,5-dion und 0,6 g 2,6-Di-tert.butyl-p-kresol in 1,4 l Xylol wird während 2 Stunden am Rückfluss erwärmt. Nach dem Erkalten wird der dunkelbraune Niederschlag von der schwarzen Reaktionslösung getrennt, in Diethylether verrührt, abgenutscht und mit Diethylether gewaschen. Durch fraktionierte Kristallisation aus Acetonitril erhält man zuerst die Titelverbindung als hellgelbe Kristalle mit einem Smp. von 177-180°C und als zweites Produkt die diastereomere Verbindung endo-1-Brom-(4-chlorphenyl)-3-(4-methoxybenzyl)-3-azabicyclo[3.1.1]heptan-2,4-dion mit einem Smp. von 142,5-143,5°C.

e) 4-Aza-2-brom-7-(4-chlorphenyl)-4-(4-methoxybenzyl)-1,6-heptadien-3,5-dion

Zu einer gerührten Suspension von 100 g Phosphorpentachlorid in 2 l Benzol werden unter Rühren 131,6 g N-(4-Methoxybenzyl)-4-chlorzimtsäureamid gegeben. Es wird 30 Minuten bei Raumtemperatur und anschliessend 30 Minuten bei 50°C nachgerührt. Nach dem Einengen wird das dunkelbraune Oel in 600 ml Toluol aufgenommen, vermischt und eingeengt. Der Rückstand wird in 1,2 l Tetrachlorkohlenstoff gelöst, mit HYFLO-Super-Cel® versetzt, abfiltriert, eingedampft und am Vakuum getrocknet. Das so erhaltene orange kristalline Zwischenprodukt wird in 1,2 l Methylenchlorid gelöst und zu einer Lösung von 430 ml 1 normaler wässriger Natriumhydrogencarbonatlösung, 6,2 g Tetra-n-butyl-ammoniumbromid und 600 ml Wasser getropft. Nach beendeter Zugabe wird 100 ml 1 normale Natriumhydrogencarbonatlösung zugetropft und während 2 Stunden weitergerührt. Die organische Phase wird abgetrennt, und die wässrige Phase wird zweimal mit Methylenchlorid extrahiert. Die vereinigten organischen Phasen werden mit Magnesiumsulfat getrocknet, eingeengt, und das Rohprodukt am Hochvakuum getrocknet. Die Titelverbindung fällt als dunkelbraunes Oel an und wird sofort gemäss 2d) weiter umgesetzt.

Beispiel 7:

a) endo-6-(4-Chlorphenyl)-3-azabicyclo[3.1.1]heptan-hydrochlorid

Analog Beispiel 1a) werden 5,9 g endo-6-(4-Chlorphenyl)-3-azabicyclo[3.1.1]heptan-2,4-dion in 130 ml Toluol mit 24 ml Natriumdihydro-bis-(2-methoxyethoxy)-aluminat-Toluollösung (70 %; FLUKA) umgesetzt und mit 24 ml konzentrierter Natronlauge aufgearbeitet. Man erhält die Titelverbindung als weisse Kristalle mit einem Smp. von 236-238°C.

Herstellung des Ausgangsmaterials

b) endo-6-(4-Chlorphenyl)-3-azabicyclo[3.1.1]heptan-2,4-dion

Zu einer gerührten Suspension von 47,7 g endo-6-(4-Chlorphenyl)-3-(4-methoxybenzyl)-3-azabicyclo[3.1.1]heptan-2,4-dion in 0.5 l Acetonitril wird bei Raumtemperatur eine Lösung von 280 g Cer(IV)-ammoniumnitrat in 390 ml Wasser zugetropft. Man rührt nach beendeter Zugabe noch 4 Stunden bei Raumtemperatur, destilliert am Wasserstrahlvakuum 200 ml Acetonitril aus dem Reaktionsgemisch ab und verdünnt dann mit 800 ml Wasser. Es wird während 1 Stunde im Eisbad gerührt, abgenutscht, und die so erhaltenen hellgelben Kristalle werden mit Wasser und Diethylether gewaschen. Das Zwischenprodukt wird in 1 l Methylenchlorid gelöst und mit 6,8 g n-Propylamin versetzt und über Nacht stehen gelassen. Anschliessend wird filtriert und die hellbraune Lösung auf 70 ml eingeengt. Es wird mit 70 ml Diethylether verdünnt, abgenutscht, und die so erhaltene graubraune kristalline Titelverbindung wird mit 50 ml Methylenchlorid-Diethylether (1:1) gewaschen; Smp. 227-228°C.

c) endo-6-(4-Chlorphenyl)-3-(4-methoxybenzyl)-3-azabicyclo[3.1.1]heptan-2,4-dion

Zu einer Lösung von 72 g endo-1-Brom-6-(4-chlorphenyl)-3-(4-methoxybenzyl)-3-azabicyclo[3.1.1]heptan-2,4-dion (Herstellung in 6d)) und 1,65 g Bisazoisobutyronitril in 1,66 l absolutem Tetrahydrofuran werden unter Stickstoffatmosphäre 49 g Tri-n-butylzinnhydrid gegeben. Die Reaktionslösung wird 1 Stunde am Rückfluss erwärmt und anschliessend eingeengt. Die so erhaltenen Kristalle werden gemäss 2b) aufgearbeitet und man erhält die Titelverbindung als weisse Kristalle mit einem Smp. von 156-158°C.

Beispiel 8:

exo-6-(4-Chlorphenyl)-3-(4-methoxybenzyl)-3-azabicyclo[3.1.1]heptan-hydrochlorid

Analog Beispiel 1a) werden 4,3 g exo-6-(4-Chlorphenyl)-3-(4-methoxybenzyl)-3-azabicyclo[3.1.1]heptan-2,4-dion in 100 ml Toluol mit 20,5 ml Natriumdihydro-bis-(2-methoxyethoxy)-aluminat-Toluollösung (70 %; FLUKA) umgesetzt und mit 5 ml konzentrierter Natronlauge aufgearbeitet. Man erhält die Titelverbindung als farblose Kristalle mit einem Smp. von 232-234°C (Zersetzung).

Herstellung der Ausgangsverbindungen

b) exo-6-(4-Chlorphenyl)-3-(4-methoxybenzyl)-3-azabicyclo[3.1.1]heptan-2,4-dion

Zu 43,5 g eines ca. 1:1-Gemisches von exo- und endo-1-Brom-6-(4-chlorphenyl)-3-(4-methoxybenzyl)-3-azabicyclo[3.1.1]heptan-2,4-dion (s. Herstellung im Beispiel 6d)) und 5 g Bisazoisobutyronitril in 1 l absolutem Tetrahydrofuran werden unter Stickstoffatmosphäre 30 g Tri-n-butylzinnhydrid gegeben. Die Reaktionslösung wird 1 Stunde am Rückfluss erwärmt und beim Erkalten auf Raumtemperatur fällt die kristalline exo-Titelverbindung aus, welche mit Diethylether gewaschen wird: Smp. 96°C.

Beispiel 9: endo-6-(4-Chlorphenyl)-3-(4-methoxybenzyl)-3-azabicyclo[3.1.1]heptan-hydrochlorid

a) Analog Beispiel 1a) werden 2 g endo-6-(4-Chlorphenyl)-3-(4-methoxybenzyl)-3-azabicyclo[3.1.1]heptan-2,4-dion (s. Herstellung in 7c)) in 50 ml Toluol mit 5 ml Natriumdihydro-bis-(2-methoxyethoxy)-aluminat-Toluollösung (70 %; FLUKA) umgesetzt und mit 5 ml konzentrierter Natronlauge aufgearbeitet. Man erhält die Titelverbindung als weisse Kristalle mit einem Smp. von 196-198°C (Zersetzung).

Beispiel 10: 1,5-Dimethyl-3-azabicyclo[3.1.1]heptan-hydrochlorid

a)Analog Beispiel 1a) werden 14 g 1,5-Dimethyl-3-azabicyclo[3.1.1.]heptan-2,4-dion in 300 ml Toluol mit 80 ml Natriumdihydro-bis-(2-methoxyethoxy)-aluminat-Toluollösung (70 %; FLUKA) umgesetzt und mit 80 ml konzentrierter Natronlauge aufgearbeitet. Man erhält die Titelverbindung als farblose Kristalle mit einem Smp. von 144,5-145,5°C.

Herstellung der Ausgangsverbindungen

b) 1,5-Dimethyl-3-azabicyclo[3.1.1]heptan-2,4-dion

Die Lösung von 47,6 g 1,5-Dimethyl-3-tert.butyl-3-azabicyclo[3.1.1]heptan-2,4-dion in 215 ml Trifluoressigsäure wird während 6 Stunden am Rückfluss erwärmt. Die eingedampfte Reaktionsmischung wird in Diethylether aufgenommen, und das kristallin ausgefallene Produkt wird filtriert und mit Diethylether gewaschen. Man erhält so die Titelverbindung mit einem Smp. von 195-196°C.

c) 1,5-Dimethyl-3-tert.butyl-3-azabicyclo[3.1.1]heptan-2,4-dion

Eine Lösung von 52,2 g 2,6-Dimethyl-4-tert.butyl-1,6-heptadien-3,5-dion und 0,5 g 2,6-Di-tert.butyl-p-kresol in 3.9 l Methylenchlorid wird während 30 Stunden analog zu 2b) bestrahlt. Nach dem Einengen wird das Rohprodukt mit Toluol-Diethylether (15:1) an 3 kg Kieselgel 60 chromatographiert. Man erhält die Titelverbindung als weisse Kristalle mit einem Smp. von 65-65,5°C (Umkristallisation bei n-Pentan bei -70°C).

d) 2,6-Dimethyl-4-tert.butyl-4-aza-1,6-heptadien-3,5-dion

Zu einer gerührten, auf 0.5° C gekühlten Lösung von 71 g N-tert.-Butyl-methacrylamid, 50,8 g Triethylamin und 570 ml Methylenchlorid wird eine Lösung von 52,7 g Methacrylsäurechlorid in 570 ml Methylenchlorid getropft. Nach der Zugabe wird 2 1/2 Stunden gerührt und die Reaktionslösung 4 Tage stehen gelassen. Das eingeengte Reaktionsgut wird in 0,5 l Diethylether aufgenommen, filtriert und die Lösung eingeengt. Das erhaltene rote Oel wird mit Hexan-Diethylether (4:1) an Kieselgel filtriert und man erhält so die Titelverbindung als weisses, kristallines Produkt (Smp. 47-48° C).

Anwendungsbeispiel

0,1 Mol 3-[6-endo(p-Chlorphenyl)-3-azabicyclo[3.1.1]-hept-3-yl]propionsäurehydrochlorid werden mit 13,4 ml 85%-iger Phosphorsäure und 50 ml Chlorbenzol unter Rühren und Rückfluss auf 100° C erhitzt. Dann werden bei 100° C 27 ml Phosphortrichlorid zugetropft, wobei Gasentwicklung stattfindet. Das Reaktionsgemisch scheidet im Lauf von 30 Minuten eine dicke Masse ab. Man erhitzt noch 3 Stunden auf 100° C und dekantiert dann das überstehende Chlorbenzol ab. Die zurückbleibende zähe Masse wird mit 100 ml 9-n Chlorwasserstoffsäure 3 Stunden unter Rühren und Rückfluss zum Sieden erhitzt. Man filtriert heiss unter Kohlezusatz und verdünnt das Filtrat mit Aceton, wobei sich die 3-[6-endo(p-Chlorphenyl)-3-azabicyclo[3.1.1]hept-3-yl]-1-hydroxy-propan-1,1-diphosphonsäure abscheidet; Smp. 224° C (Zersetzung).

Tabletten, enthaltend 75 mg des Wirkstoffs, oder ein Salz, z.B. das Natriumsalz, davon, können folgendermassen hergestellt werden:

Bestandteile (für 1000 Tabletten)

| | | |
|---|---|---|
| Wirkstoff | | 75,0 g |
| Lactose | | 268,5 g |
| Maisstärke | | 22,5 g |
| Polyäthylenglykol 6000 | | 5,0 g |
| Talkum | | 15,0 g |
| Magnesiumstearat | | 4,0 g |
| entmineralisiertes Wasser | q.s. | |

Herstellung:

Die festen Ingredienzien werden zunächst durch ein Sieb mit 0,6 mm Maschenweite getrieben. Dann wird der Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyäthylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° C getrocknet, durch ein Sieb mit 1,2 mm Maschenweite getrieben und zu beidseitig konkaven Tabletten von etwa 10 mm Durchmesser mit Bruchkerbe auf der Oberseite verpresst.

**Patentansprüche**

1. Verbindungen der Formel I

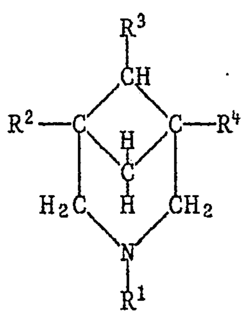

(I)

und ihre Ammoniumsalze, worin

$R^1$ ein Wasserstoffatom, lineares oder verzweigtes $C_1$-$C_{20}$-Alkyl, $C_3$-$C_8$-Cycloalkyl, $C_4$-$C_{20}$-Alkylcycloal-

kyl, $C_4$-$C_{12}$-Cycloalkylalkyl, $C_5$-$C_{20}$-Alkylcycloalkylalkyl, $C_6$-$C_{14}$-Aryl, $C_7$-$C_{20}$-Alkaryl oder -Aralkyl oder $C_8$-$C_{20}$-Alkaralkyl bedeutet, das unsubstituiert oder mit Halogen, -OH, -CN, -$NO_2$, $C_1$-$C_6$-Halogenalkyl, -COOH, -$SO_3H$, $R^5CO$-, $R^5COO$-, $R^5OCO$-, $R^5OSO_2$-, $R^5SO_2$-, $R^5SO_3$-, $C_1$-$C_6$-Halogenalkyl, $C_1$-$C_6$-Alkoxy, $C_1$-$C_6$-Alkylthio, $C_6$-$C_{10}$-Aryloxy oder -Arylthio, $C_7$-$C_{12}$-Aralkyloxy oder - Aralkylthio substituiert ist, wobei $R^5$ $C_1$-$C_{12}$-Alkyl, $C_4$-$C_7$-Cycloalkyl, $C_5$-$C_{16}$-Alkylcycloalkyl oder -Cycloalkylalkyl, $C_6$-$C_{16}$-Alkylcycloalkylalkyl, $C_6$-$C_{10}$-Aryl, $C_7$-$C_{16}$-Aralkyl oder -Alkaryl oder $C_8$-$C_{16}$-Alkaralkyl ist,

$R^2$ für ein Wasserstoffatom, lineares oder verzweigtes $C_1$-$C_{20}$-Alkyl, -Alkoxy, -Alkylthio, $C_3$-$C_8$-Cycloalkyl, $C_4$-$C_{20}$-Alkylcycloalkyl oder -Cycloalkylalkyl, $C_5$-$C_{20}$-Alkylcycloalkylalkyl, $C_3$-$C_8$-Cycloalkyloxy oder -thio, $C_4$-$C_{20}$-Alkylcycloalkyloxy oder -thio oder -Cycloalkylalkyloxy oder -thio, $C_5$-$C_{20}$-Alkylcycloalkylalkyloxy oder -thio, $C_6$-$C_{14}$-Aryl, -Aryloxy oder -thio, $C_7$-$C_{20}$-Alkaryl, -Alkaryloxy oder -thio, $C_7$-$C_{20}$-Aralkyl, $C_7$-$C_{20}$-Aralkyloxy oder -thio, $C_8$-$C_{20}$-Alkaralkyl, $C_8$-$C_{20}$-Alkaralkyloxy oder -thio steht, das unsubstituiert oder wie für $R^1$ definiert, substituiert ist,

$R^4$ unabhängig die gleiche Bedeutung wie $R^2$ hat mit Ausnahme von unsubstituiertem, oder wie für $R^1$ definiert substituiertem $C_6$-$C_{14}$-Aryl und $C_7$-$C_{20}$-Alkaryl, und

$R^3$ unabhängig die Bedeutung von $R^2$ hat, oder Halogen, Carboxyl oder Carboxylat mit 2 bis 16 C-Atomen darstellt, mit der Massgabe, dass $R^1$, $R^2$, $R^3$ und $R^4$ nicht gleichzeitig für H stehen.

2. Verbindungen gemäss Anspruch 1, worin $R^1$ ein Wasserstoffatom oder unsubstituiertes oder substituiertes $C_1$-$C_{12}$-Alkyl, $C_5$-$C_7$-Cycloalkyl, $C_6$-$C_{16}$-Alkylcycloalkyl oder -Cycloalkylalkyl, $C_7$-$C_{16}$-Alkylcycloalkylalkyl, $C_6$-$C_{10}$-Aryl, $C_7$-$C_{16}$-Aralkyl oder -Alkaryl oder $C_8$-$C_{16}$-Alkaralkyl bedeutet.

3. Verbindungen gemäss Anspruch 1, worin $R^1$ ein Wasserstoffatom oder unsubstituiertes oder substituiertes $C_1$-$C_6$-Alkyl, Cyclopentyl, Cyclohexyl, $C_1$-$C_4$-Alkylcyclopentyl oder -cyclohexyl, Cyclopentyl-$C_nH_{2n}$- oder Cyclohexyl-$C_nH_{2n}$-, $C_1$-$C_4$-Alkylcyclopentyl-$C_nH_{2n}$- oder $C_1$-$C_4$-Alkylcyclohexyl-$C_nH_{2n}$-, Phenyl, $C_1$-$C_4$-Alkylphenyl, Phenyl-$C_nH_{2n}$-, $C_1$-$C_4$-Alkylphenyl-$C_nH_{2n}$- darstellt, wobei n eine Zahl von 1 bis 4 ist.

4. Verbindungen gemäss Anspruch 1 worin $R^1$ H, unsubstituiertes oder substituiertes $C_1$-$C_6$-Alkyl, Phenyl oder Benzyl darstellt.

5. Verbindungen gemäss Anspruch 1, worin $R^2$ für H steht, oder unsubstituiertes oder substituiertes $C_1$-$C_{12}$-Alkyl, -Alkoxy oder -Alkylthio, $C_5$-$C_7$-Cycloalkyl, $C_6$-$C_{16}$-Alkylcycloalkyl oder -Cycloalkylalkyl, $C_7$-$C_{16}$-Alkylcycloalkylalkyl, $C_5$-$C_7$-Cycloalkoxy oder -thio, $C_6$-$C_{16}$-Alkylcycloalkyloxy oder -thio oder -Cycloalkylalkyloxy oder -thio, $C_7$-$C_{16}$-Alkylcycloalkylalkyloxy oder -thio, $C_6$-$C_{10}$-Aryl, -Aryloxy oder -thio, $C_7$-$C_{16}$-Alkaryl, -Alkaryloxy oder -thio oder -Aralkyloxy oder -thio, $C_8$-$C_{16}$-Alkaralkyloxy oder -thio, $C_7$-$C_{16}$-Alkaryl oder $C_8$-$C_{16}$-Alkaralkyl darstellt.

6. Verbindungen gemäss Anspruch 1, worin $R^2$ für ein Wasserstoffatom steht oder unsubstituiertes oder substituiertes $C_1$-$C_6$-Alkyl, Phenyl oder $C_1$-$C_4$-Alkylphenyl darstellt.

7. Verbindungen gemäss Anspruch 1, worin $R^2$ für H, $C_1$-$C_4$-Alkyl, unsubstituiertes oder substituiertes Phenyl oder $C_1$-$C_4$-Alkylphenyl steht.

8. Verbindungen gemäss Anspruch 1, worin $R^4$ für H oder $C_1$-$C_6$-Alkyl steht.

9. Verbindungen gemäss Anspruch 1, worin $R^3$ für H steht, oder unsubstituiertes oder substituiertes $C_1$-$C_{12}$-Alkyl, -Alkoxy oder -Alkylthio, $C_5$-$C_7$-Cycloalkyl, $C_6$-$C_{16}$-Alkylcycloalkyl oder -Cycloalkylalkyl, $C_7$-$C_{16}$-Alkylcycloalkylalkyl, $C_5$-$C_7$-Cycloalkoxy oder -thio, $C_6$-$C_{16}$-Alkylcycloalkyloxy oder -thio oder -Cycloalkylalkyloxy oder -thio, $C_7$-$C_{16}$-Alkylcycloalkylalkyloxy oder -thio, $C_6$-$C_{10}$-Aryl, -Aryloxy oder -thio, $C_7$-$C_{16}$-Alkaryl, -Alkaryloxy oder -thio oder -Aralkyloxy oder -thio, $C_8$-$C_{16}$-Alkaralkyloxy oder -thio, $C_7$-$C_{16}$-Alkaryl oder $C_8$-$C_{16}$-Alkaralkyl, F, Cl, Br, Carboxyl oder Carboxylat mit 2 bis 8 C-Atomen darstellt.

10. Verbindungen gemäss Anspruch 1, worin $R^3$ H, $C_1$-$C_6$-Alkyl, unsubstituiertes oder substituiertes Phenyl oder $C_1$-$C_4$-Alkylphenyl ist.

11. Verbindungen gemäss Anspruch 1, worin $R^4$ H und $R^2$ Phenyl, oder $R^2$ und $R^4$ Methyl bedeuten und $R^1$ und $R^3$ für H stehen.

12. Verfahren zur Herstellung von Verbindungen der Formel I gemäss Anspruch 1, dadurch gekennzeichnet, dass man eine Verbindung der Formel II

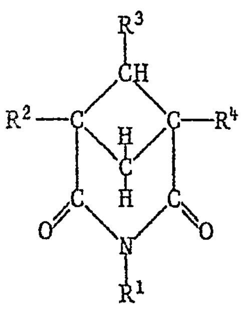

(II),

worin $R^1$, $R^2$, $R^3$ und $R^4$ die in Anspruch 1 angegebenen Bedeutungen haben, reduziert.

13. Verfahren gemäss Anspruch 2, dadurch gekennzeichnet, dass man die Reduktion mit einem bisalkoxylierten Natriumdihydroaluminat durchführt.

14. Verfahren gemäss Anspruch 13, dadurch gekennzeichnet, dass die Reduktion mit Natriumdihydro-bis-(2-methoxyethoxy)-aluminat durchgeführt wird.